# EUROPÄISCHE PATENTANMELDUNG

(11) **EP 3 854 421 A1**
(43) Veröffentlichungstag der Anmeldung: **28.07.2021**
(21) Anmeldenummer: 20020418.8
(22) Anmeldetag: 17.09.2020
(51) Int. Cl.: A61L 2/10, A61L 2/22, A61L 2/23, B24C 1/00

(54) **TROCKENEIS-PELLETS MIT DESINFEKTIONSWIRKUNG**

(30) Priorität: 27.01.2020 DE 102020000465
(71) Anmelder: Linde GmbH, 82049 Pullach (DE)
(72) Erfinder: Blanke, Martin, 85521 Riemerling (DE); Glück, Robert, 82178 Puchheim (DE)
(74) Vertreter: Lu, Jing

(57) **Zusammenfassung**

Die Erfindung betrifft ein Verfahren zum Reinigen und Desinfizieren eines Gegenstands (10), aufweisend die Schritte: Erzeugen eines Stoffstromes (S) aufweisend Trockeneis (T) und ein Trägergas (G), und Ausstoßen des Stoffstromes (S) aus einer Düse (2), so dass der Stoffstrom (S) auf eine Oberfläche (11) des Gegenstands (10) auftrifft, wobei die Oberfläche (11) mittels des Trockeneises (T) gereinigt wird, und wobei die Oberfläche mittels eines Mittels zum Desinfizieren (D, 8) desinfiziert wird. Bei dem Mittel zum Desinfizieren kann es sich z.B. um ein Desinfektionsmittel (D) handeln, das ein Bestandteil des Stoffstromes (S) ist, oder um UV-Licht (8), das mittels zumindest einer UV-Lampe (7) erzeugt wird.

## Beschreibung

Die Erfindung betrifft ein Verfahren und eine Vorrichtung zum Reinigen und Desinfizieren von Gegenständen, insbesondere von Krankenhausmobiliar und Einrichtungsgegenständen, insbesondere Krankenhausbetten.

Im Bereich der Medizin und der Physiotherapie ist es üblich, die verwendeten Arbeitsmittel zu reinigen und zu desinfizieren bzw. zu sterilisieren. Grundsätzlich sind dies drei verschiedene Vorgänge mit unterschiedlicher Aufgabenstellung und Zielsetzung. Der Reinigungsvorgang entfernt oberflächliche Anhaftungen. Hierfür kommen vorzugsweise nasse Reinigungsverfahren auf Basis von Seifen oder organischen Fluiden zum Einsatz. Desinfektion soll keimfähige Verunreinigungen so behandeln, dass eine Infektion nicht mehr erfolgen kann. Sterilisation ist eine höhere Form der Desinfektion und reduziert die Zahl der Keime um weitere Potenzen. Bei der Desinfektion unterscheidet man thermische, chemische, sowie UV-Desinfektion, Plasma- und Membran-Desinfektion.

Wie oben dargelegt, stellen die Reinigung und Desinfektion eines Gegenstandes zwei voneinander unabhängige Schritte dar. Werden hierbei wässrige bzw. flüssige Reinigungsmittel verwendet, ist das zu reinigende Gut bzw. der zu reinigende Gegenstand anschließend nass. Das kann unter Umständen Schäden an dem Gegenstand hervorrufen, insbesondere wenn dieser Funktionskomponenten (z.B. elektronische Komponenten) und/oder Materialien aufweist, die nicht in übermäßigen Kontakt mit einer Flüssigkeit gelangen sollen. Darüber hinaus können organische Substanzen und Lösemittel (VOC) negative Effekte auf die Umwelt zur Folge haben. Wird eine keimreduzierende Wirkung oder Desinfektion gefordert, sind in der Regel weitere Schritte gefordert.

Der Erfindung liegt hiervon ausgehend die Aufgabe zugrunde, ein verbessertes Verfahren und eine entsprechende Vorrichtung bereitzustellen, das bzw. die ein effektives Reinigen und Desinfizieren eines Gegenstandes in einem Arbeitsschritt erlauben.

Diese Aufgabe wird durch ein Verfahren mit den Merkmalen des Anspruchs 1 und durch eine Vorrichtung mit den Merkmalen des Anspruchs 15 gelöst. Bevorzugte Ausgestaltungen des jeweiligen Erfindungsaspekt werden nachfolgend beschrieben.

Gemäß Anspruch 1 wird ein Verfahren zum Reinigen und Desinfizieren eines Gegenstands offenbart, wobei das Verfahren die Schritte aufweist:
- Erzeugen eines Stoffstromes aufweisend Trockeneis und ein Trägergas, und
- Ausstoßen des Stoffstromes aus einer Düse, so dass der Stoffstrom auf eine Oberfläche des Gegenstands auftrifft, wobei die Oberfläche mittels des Trockeneises gereinigt wird, und
- Desinfizieren der Oberfläche mittels eines Mittels zum Desinfizieren.

Die Düse kann dabei in eine Strahlpistole integriert sein. Die Strahlpistole kann einen Handgriff aufweisen, so dass eine Person die Strahlpistole greifen und führen kann, um die Düse bzw. Strahlpistole auf eine zu reinigende bzw. desinfizierende Oberfläche zu richten.

Gemäß einer bevorzugten Ausführungsform ist das Mittel zum Desinfizieren durch ein Desinfektionsmittel gebildet, wobei der Stoffstrom das Desinfektionsmittel aufweist, so dass die Oberfläche mittels des Trockeneises gereinigt wird und mittels des Desinfektionsmittels desinfiziert wird, wenn der Stoffstrom auf die Oberfläche auftrifft.

Es ist bekannt, dass CO₂ eine keimhemmende, aber keine desinfizierende Wirkung hat. Aufgrund der erfindungsgemäßen Zugabe des Desinfektionsmittels wird dieser Umstand effizient verbessert.

Hierbei kann das Trockeneis in Form von Trockeneisschnee vor oder in Form einer Vielzahl an Trockeneiskörpern vorliegen, die vorzugsweise die Form von längserstreckten Trockeneiskörpern bzw. Pellets aufweisen, die vorzugsweise jeweils einen Durchmesser aufweisen, der kleiner oder gleich 3 mm ist.

Mittels des Trockeneises (insbesondere in Form von Trockeneisschnee bzw. Trockeneispartikeln oder Trockeneiskörpern/Pellets) kann die Oberfläche des zu prozessierenden Gegenstandes effizient nicht abrasiv von der Verschmutzung befreit werden, wobei gleichzeitig aufgrund des ebenfalls ausgebrachten Desinfektionsmittels ein Desinfizieren des Gegenstandes mit Vorteil in einem Arbeitsschritt möglich ist.

Beim Trockeneis handelt es sich um in den festen Aggregatzustand überführtes und auf mindestens -78,5 °C gekühltes Kohlendioxid (CO₂). Trockeneis geht unter Atmosphärendruck unmittelbar vom festen Aggregatzustand in den gasförmigen Aggregatzustand über, wobei keine Schmelzflüssigkeit entsteht. Dadurch kann auf besonders einfache Weise mittels des Trägergases sowohl der Beschuss mit den Trockeneispartikeln als auch die Absaugung und Abfuhr der Schmutzpartikel erfolgen.

Durch den mittels der Düse ausgegebenen Stoffstrom können auch schlecht zugängliche Bereiche des Gegenstandes, wie z. B. Hinterschneidungen oder Ecken und Kanten der Oberfläche des Gegenstandes, gut erreicht werden. Flüssige (chemische) Desinfektionsmittel auf Basis von Alkoholen sind zudem gut geeignet, um die Verschmutzung zu lösen, die im Anschluss leichter entfernt werden kann.

Zum Beschleunigen der Trockeneispartikel handelt es sich bei der Düse vorzugsweise um eine Lavaldüse. Es kann jedoch auch z.B. eine Venturidüse eingesetzt werden.

Gemäß einer bevorzugten Ausführungsform der Erfindung ist vorgesehen, dass das Desinfektionsmittel einen der folgenden Stoffe aufweist oder durch einen der folgenden Stoffe gebildet ist: Methanol (Gefrierpunkt -97,60 °C), Ethanol (Gefrierpunkt -114,10 °C), Propanol (Gefrierpunkt -126,20 °C), Butanol (Gefrierpunkt -89,30 °C), Pentanol (Gefrierpunkt -77,60 °C), Hexanol (Gefrierpunkt -44,60 °C), Heptanol (Gefrierpunkt - 34,00 °C), Oktanol (Gefrierpunkt -15,50 °C), Isopropanol (Gefrierpunkt -87,90 °C), Isobutanol (Gefrierpunkt -108,00 °C), Isopentanol (Gefrierpunkt -117,20 °C), Ethylenglykol (Gefrierpunkt -13,00 °C), 1,3-Propylenglykol (Gefrierpunkt -26,70 °C).

Das Desinfektionsmittel kann auch durch ein beliebiges Gemisch der oben genannten Stoffe gebildet sein, oder weitere desinfizierende organische Stoffe mit ähnlichen Eigenschaften beinhalten.

Weiterhin ist gemäß einer bevorzugten Ausführungsform des Verfahrens vorgesehen, dass das Desinfektionsmittel (bezogen auf Normalbedingungen) einen Gefrierpunkt aufweist, der vorzugsweise unterhalb von 0°C, vorzugsweise unterhalb von -78,5° C liegt. Hierdurch können mit Vorteil Vereisungen des Desinfektionsmittels verhindert werden.

Weiterhin ist gemäß einer bevorzugten Ausführungsform des Verfahrens vorgesehen, dass das Trockeneis in Form einer Vielzahl an festen Trockeneiskörpern in das Trägergas gegeben wird. Bei den Trockeneiskörpern kann es sich z. B. um Mikropartikel handeln. Die Trockeneiskörper können z. B. einen Durchmesser aufweisen, der kleiner oder gleich 3 mm ist. Vorzugsweise weisen die Trockeneiskörper jeweils einen Durchmesser im Bereich von 0,1 mm bis 0,5 mm auf. Bei den Trockeneiskörpern kann es sich auch um größere Trockeneiskörper handeln, insbesondere in Form von längserstreckten Trockeneiskörpern (sogenannte Pellets), die vorzugsweise jeweils ein Volumen im Bereich von 0,5 mm³ bis 10 mm³ aufweisen.

Weiterhin besteht die Möglichkeit, das Trockeneis in Form von Trockeneisschnee in das Trägergas zu geben.

Insbesondere kann das Kohlendioxid in flüssiger Form vorgehalten werden, wobei das flüssige Kohlendioxid vorzugsweise in einer Expansionskammer unter Entstehung von Trockeneisschnee expandiert wird und sodann in das Trägergas gegeben wird. Bei dieser Ausführungsform wird also insbesondere das Trockeneis frisch kurz vor dem Einbringen in das Trägergas erzeugt.

Gemäß einer bevorzugten Ausführungsform des erfindungsgemäßen Verfahrens ist weiterhin vorgesehen, dass das Desinfektionsmittel im flüssigen Zustand an einer Einspeisestelle in das Trägergas gegeben wird.

Weiterhin ist gemäß einer bevorzugten Ausführungsform des Verfahrens vorgesehen, dass das Trockeneis zur Erzeugung des besagten Stoffstromes (aufweisend das Trägergas, das Desinfektionsmittel und die Trockeneispartikel) - bezogen auf eine Strömungsrichtung des Trägergases - stromab der Einspeisestelle in das Trägergas gegeben wird. Alternativ hierzu ist vorgesehen, dass das Trockeneis - bezogen auf eine Strömungsrichtung des Trägergases - stromauf der Einspeisestelle in das Trägergas gegeben wird.

Gemäß einer weiteren bevorzugten Ausführungsform ist vorgesehen, dass eine Mischung (bzw. der besagte Stoffstrom) aus dem Trockeneis (insbesondere Trockeneispartikel), Desinfektionsmittel und Trägergas in einer Düse in Form einer Lavaldüse beschleunigt wird bevor die Mischung bzw. der Stoffstrom auf die zu reinigende Oberfläche auftrifft.

Weiterhin ist gemäß einer bevorzugten Ausführungsform der Erfindung vorgesehen, dass das Desinfektionsmittel über ein Drosselventil und eine Venturidüse in das Trägergas gegeben wird. Hierbei kann das Desinfektionsmittel über eine Zuleitung in eine Engstelle der Venturidüse gegeben werden.

Gemäß einer alternativen bevorzugten Ausführungsform der Erfindung kann vorgesehen sein, dass das Desinfektionsmittel dem Trockeneis zugegeben wird, bevor das Trockeneis in das Trägergas gegeben wird.

Insbesondere kann das Verfahren vorsehen, dass zumindest ein Trockeneisblock aufweisend das Desinfektionsmittel bereitgestellt wird, wobei der Trockeneisblock zur Erzeugung der Trockeneiskörper aus Trockeneis zerkleinert wird (z.B. mittels eines Mahlwerks oder eines sonstigen Zerkleinerers). Insbesondere kann zum Herstellen des mindestens einen Trockeneisblocks das Desinfektionsmittel mit Trockeneiskörpern, insbesondere Trockeneispellets vermischt werden, die dann zu zumindest einem Trockeneisblock gepresst werden. Der bzw. die gepressten Trockeneisblöcke können dann z.B. in kleinen oder mittleren Strahlanlagen mit Mahlwerk bzw. Zerkleinerer verstrahlt werden.

Weiterhin ist gemäß einer bevorzugten Ausführungsform des erfindungsgemäßen Verfahrens vorgesehen, dass dem Stoffstrom eine Menge an Desinfektionsmittel zugegeben wird, so dass die Konzentration des Desinfektionsmittels im Trägergas im Bereich von 10 g/m³ bis 250 g/m³ liegt, vorzugsweise im Bereich von 40 g/m³ bis 100 g/m³

Gerade bei niedrigen Strahldrücken des ausgegebenen Stoffstroms, die z.B. unterhalb von 4 bar, insbesondere unterhalb von 3 bar liegen, verbleibt somit genügend Flüssigkeit des Desinfektionsmittels auf der Oberfläche des Gegenstands um auch nach dem Reinigungsgang einen langanhaltenden Desinfektionseffekt zu bewirken.

Gemäß einer bevorzugten Ausführungsform des erfindungsgemäßen Verfahrens kann die Düse bzw. eine Strahlpistole, in die die Düse integriert ist, manuell von einem Bediener oder automatisch von einem Roboter oder einer entsprechenden Vorrichtung geführt werden.

Weiterhin ist gemäß einer bevorzugten Ausführungsform des erfindungsgemäßen Verfahrens vorgesehen, dass das Mittel zum Desinfizieren UV-Licht ist. Ultraviolettes (UV) Licht wird im Sinne der vorliegenden Erfindung insbesondere als Licht verstanden, dass eine Wellenlänge im Bereich von 100 nm bis 380 nm, vorzugsweise 240 nm bis 270 nm aufweist.

Bekanntermaßen hängt die Wirksamkeit des UV-Lichtes von der Strahlungsintensität ab (bei kleinem Abstand genügt eine kurze Zeit um eine für einen Keim eine letale Wirkung zu erzielen) sowie weiterhin von der Dauer der Exposition (insbesondere von wenigen Sekunden bis ca. 2 Minuten) sowie weiterhin vom bestrahlten Keim. Die jeweilige letale Dosis ergibt sich somit aus dem Produkt der Strahlungsintensität und der Bestrahlungszeit und ist für die einzelnen Keime/Bakterien oder Viren bekannt.

Bei den Ausführungsformen des Verfahrens, die UV-Licht verwenden, kann das Trockeneis in einer der hierin bereits beschriebenen Weisen bereitgestellt werden.

Insbesondere kann bei einer Verwendung von UV-Licht die zu desinfizierende Oberfläche vor und/oder während und/oder nach dem Reinigen der Oberfläche mittels des Trockeneises mit dem UV-Licht beaufschlagt werden, um die Oberfläche zu desinfizieren.

Zum Bestrahlen der Oberfläche mit UV-Licht kann zumindest eine UV-Lampe vorgesehen sein, die das UV-Licht erzeugt. Die mindestens eine UV-Lampe kann in eine die Düse aufweisende Strahlpistole integriert sein oder kann daran befestigt sein, wobei der Stoffstrom über die Düse bzw. Strahlpistole auf die Oberfläche ausgegeben wird und die Oberfläche z.B. gleichzeitig mit dem UV-Licht bestrahlbar ist. Beim manuellen Führen der Düse bzw. Strahlpistole kann somit zugleich die Oberfläche mit UV-Licht bestrahlt werden.

Es ist auch möglich, dass die UV-Lampe nicht mit der Düse starr verbunden ist und getrennt von der Düse geführt wird (z.B. manuell). Weiterhin kann eine Mehrzahl an UV-Lampen stationär um die zu reinigende Oberfläche aufgestellt sein um diese z.B. vor und/oder während und/oder nach der Beaufschlagung mit Trockeneis zu reinigen.

Bevorzugt wird das erfindungsgemäße Verfahren auf Gegenstände angewendet, bei denen eine zusätzliche Desinfizierung von großer Bedeutung ist. Insbesondere handelt es sich dabei um Einrichtungsgegenstände, die in Krankenhäusern eingesetzt werden, wie z. B. Krankenhausbetten bzw. Bettgestelle. Das erfindungsgemäße Verfahren kann natürlich auf alle erdenklichen anderen Gegenstände angewendet werden, die durch eine Beaufschlagung mit Trockeneispartikeln nicht beschädigt werden und die eine desinfizierende Behandlung benötigen bzw. erhalten sollen. Weiterhin werden das erfindungsgemäße Verfahren bzw. die erfindungsgemäße Vorrichtung gemäß einer bevorzugten Ausführungsform zur Reinigung und Desinfektion von Gasbehältern, sowohl gasförmig als auch flüssig (Niederdruck und Hochdruck), zur Anwendung im medizinischen als auch nicht-medizinischen Bereich, verwendet.

Gemäß einer bevorzugten Ausführungsform des Verfahrens ist weiterhin vorgesehen, dass das Trägergas eines der folgenden Gase ist: Luft, insbesondere Druckluft mit einem Strahldruck bis zu 10 bar, im Einzelfall bis zu 16 bar, vorzugsweise im Bereich von 2 bis 4 bar; ein inertes Gas, gasförmiges CO₂, Stickstoff.

Vorzugsweise weist das Trägergas vor dem Ausstoßen aus der Düse einen Druck im Bereich von 2 bar bis 4 bar auf.

Weiterhin wird vorzugsweise der Stoffstrom (Trockeneis, Trägergas und Desinfektionsmittel) mit einem Volumenstrom im Bereich von 0,2 m³/min bis 2 m³/min, vorzugsweise 0,3 m³/min bis 0,8 m³/min, aus der Düse ausgegeben.

Auch bei der Verwendung von UV-Licht ist gemäß einer bevorzugten Ausführungsform des erfindungsgemäßen Verfahrens vorgesehen, dass die Düse bzw. eine Strahlpistole, in die die Düse integriert ist, manuell von einem Bediener oder automatisch von einem Roboter oder einer entsprechenden Vorrichtung geführt werden.

Ein weiterer Aspekt der Erfindung betrifft eine Vorrichtung zum Reinigen und Desinfizieren eines Gegenstands, wobei die Vorrichtung dazu ausgebildet ist, einen Stoffstrom aufweisend Trockeneis und ein Trägergas bereitzustellen, wobei die Vorrichtung weiterhin eine Düse aufweist und dazu ausgebildet ist, den Stoffstrom aus der Düse zum Reinigen einer Oberfläche des Gegenstands auszustoßen, und wobei die Vorrichtung dazu ausgebildet ist ein Mittel zum Desinfizieren der Oberfläche bereitzustellen

Gemäß einer bevorzugten Ausführungsform der Vorrichtung ist das Mittel zum Desinfizieren ein Desinfektionsmittel, das der Stoffstrom aufweist (siehe z.B. oben).

Gemäß einer bevorzugten Ausführungsform der Erfindung ist vorgesehen, dass die Düse einen Endabschnitt eines Strömungspfads der Vorrichtung bildet.

Weiterhin ist gemäß einer bevorzugten Ausführungsform der Erfindung vorgesehen, dass die Vorrichtung dazu ausgebildet ist, das Trockeneis in Form einer Vielzahl an Trockeneiskörpern in das Trägergas zu geben (siehe auch oben). Z. B. kann die Vorrichtung einen Behälter zum Speichern der CO₂-Trockeneiskörper/Pellets bzw. des Trockeneises aufweisen, der mit dem Strömungspfad verbindbar ist, z.B. über eine Schleuse, so dass das Trockeneis bzw. die Trockeneiskörper/Pellets über die Schleuse in den Strömungspfad einleitbar sind und damit dem Trägergas zuführbar sind.

Gemäß einer alternativen bevorzugten Ausführungsform ist vorgesehen, dass die Vorrichtung einen Behälter zum Speichern von Kohlendioxid in flüssiger Form aufweist, wobei die Vorrichtung dazu ausgebildet ist, das flüssige Kohlendioxid unter Erzeugung von Trockeneisschnee zu expandieren (z.B. in einer Expansionskammer der Vorrichtung) und sodann dem Strömungspad bzw. dem darin strömenden Trägergas zuzugeben.

Weiterhin ist gemäß einer bevorzugten Ausführungsform der Erfindung vorgesehen, dass der Strömungspad der Vorrichtung eine Einspeisestelle aufweist, über die das Desinfektionsmittel in einem flüssigen Zustand in den Strömungspfad bzw. in das darin geführte Trägergas einleitbar ist.

Gemäß einer weiteren bevorzugten Ausführungsform der Erfindung ist vorgesehen, dass die Vorrichtung dazu ausgebildet ist, das Trockeneis - bezogen auf eine Strömungsrichtung des Trägergases - stromab der Einspeisestelle in den Strömungspfad bzw. in das darin geführte Trägergas zu geben, oder dass die Vorrichtung dazu ausgebildet ist, das Trockeneis - bezogen auf eine Strömungsrichtung des Trägergases - stromauf der Einspeisestelle in den Strömungspfad bzw. in das darin geführte Trägergas zu geben.

Weiterhin ist gemäß einer bevorzugten Ausführungsform der Erfindung vorgesehen, dass die Vorrichtung eine Venturidüse aufweist, wobei die Vorrichtung dazu ausgebildet ist, das Desinfektionsmittel über die Venturidüse in den Strömungspfad bzw. das darin geführte Trägergas zu geben. Stromauf der Engstelle (bezogen auf eine Strömungsrichtung des Desinfektionsmittels zur Venturidüse hin) kann ein Drosselventil zum Drosseln eines Volumenstroms des Desinfektionsmittels vorgesehen sein.

Weiterhin kann gemäß einer bevorzugten Ausführungsform der Vorrichtung vorgesehen sein, dass die Vorrichtung dazu ausgebildet ist, das Desinfektionsmittel dem Trockeneis zuzugeben, bevor das Trockeneis in das Trägergas gegeben wird. Insbesondere kann die Vorrichtung gemäß einer bevorzugten Ausführungsform einen Zerkleinerer aufweisen, der dazu ausgebildet ist, einen Trockeneisblock zur Herstellung der besagten Trockeneiskörper zu zerkleinern. Der Trockeneisblock weist dabei bereits das Desinfektionsmittel auf. Alternativ kann das Desinfektionsmittel vor oder nach der Zugabe von Trockeneiskörpern in das Trägergas gegeben werden.

Gemäß einer weiteren bevorzugten Ausführungsform der Erfindung ist vorgesehen, dass das Mittel zum Desinfizieren UV-Licht ist, wobei die Vorrichtung zumindest eine Lampe zum Erzeugen des UV-Lichts aufweist.

Gemäß einer Ausführungsform der Erfindung ist vorgesehen, dass die Vorrichtung eine Strahlpistole aufweist, wobei die Düse in die Strahlpistole integriert ist oder an dieser festgelegt ist, und wobei insbesondere die mindestens eine UV-Lampe an der Strahlpistole festgelegt ist.

Gemäß einer alternativen bevorzugten Ausführungsform der Vorrichtung ist vorgesehen, dass die mindestens eine UV-Lampe separat zur Düse ausgebildet ist. So kann die mindestens eine UV-Lampe zum Beispiel getrennt von der Düse führbar sein (z.B. manuell). Weiterhin kann die Vorrichtung eine Mehrzahl an separaten UV-Lampen aufweisen, die stationär um die zu reinigende Oberfläche aufstellbar sind.

Insbesondere ist gemäß einer bevorzugten Ausführungsform vorgesehen, dass die Vorrichtung in verschiedenen Richtungen strahlende stationäre UV-Lampen aufweist, die dazu ausgebildet sind, die zu desinfizierende Oberfläche anzustrahlen. Im Fall von stationären UV-Lampen ist die Düse bzw. Strahlpistole unabhängig von den UV-Lampen bezüglich er zu reinigenden Oberfläche bewegbar.

Bei den Ausführungsformen der erfindungsgemäßen Vorrichtung, die UV-Licht zum Desinfizieren der Oberfläche verwenden, kann das Trockeneis grundsätzlich in einer der oben im Zusammenhang mit dem Desinfektionsmittel beschriebenen Weisen bereitgestellt werden.

Grundsätzlich kann die Düse bzw. die Strahlpistole gemäß einer bevorzugten Ausführungsform der erfindungsgemäßen Vorrichtung manuell von einem Bediener oder automatisch von einem Roboter oder einer entsprechenden Vorrichtung geführt werden.

Gemäß einer bevorzugten Ausführungsform der Erfindung ist die Vorrichtung dazu ausgebildet, die Düse und/oder die Strahlpistole automatisch entlang einer zu reinigenden Oberfläche zu führen und derart auszurichten, dass die Oberfläche mittels des Stoffstroms reinigbar und mittels des ausgestrahlten UV-Lichts desinfizierbar ist. Hierzu kann die Vorrichtung einen Roboter, insbesondere einen Roboterarm, aufweisen.

Weitere Merkmale und Vorteile der Erfindung sollen durch die Beschreibung von Ausführungsbeispielen anhand der Figur erläutert werden. Es zeigen:
Fig. 1 eine schematische Ansicht einer Ausführungsform einer erfindungsgemäßen Vorrichtung bzw. eines erfindungsgemäßen Verfahrens zum Reinigen und Desinfizieren einer Oberfläche eines Gegenstandes; und
Fig. 2 eine schematische Ansicht einer weiteren Ausführungsform einer erfindungsgemäßen Vorrichtung bzw. eines erfindungsgemäßen Verfahrens zum Reinigen und Desinfizieren einer Oberfläche eines Gegenstandes

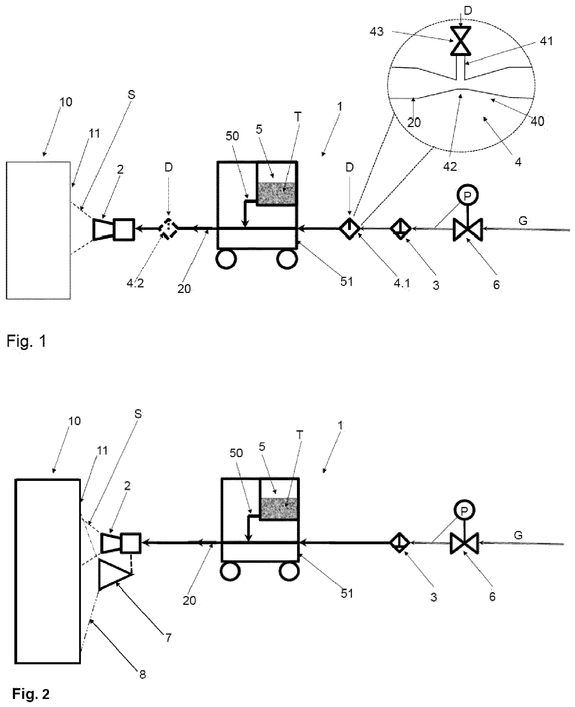

Die Figur 1 zeigt eine Ausführungsform einer erfindungsgemäßen Vorrichtung 1 die zur Durchführung des erfindungsgemäßen Verfahrens ausgebildet ist. Bei dem Verfahren wird zum Reinigen und Desinfizieren eines Gegenstands 10 ein Stoffstrom S erzeugt, der Trockeneis T, ein Trägergas G sowie ein Desinfektionsmittel D aufweist, wobei das Trägergas (z.B. Druckluft) zum Beschleunigen des Trockeneises bzw. des Desinfektionsmittels D verwendet wird. Dieser Stoffstrom S wird aus einer Düse 2 der Vorrichtung 1 ausgestoßen, die z.B. als Lavaldüse ausgebildet sein kann. Der Stoffstrom S trifft sodann auf eine Oberfläche 11 des Gegenstands 10, wobei die Oberfläche 11 mittels des darauf auftreffenden Trockeneises T gereinigt wird und mittels des mitgeführten Desinfektionsmittels D desinfiziert wird. Das Desinfektionsmittel kann die oben angegebenen Stoffe aufweisen bzw. kann durch einen dieser Stoffe gebildet sein und weist insbesondere einen Gefrierpunkt auf, der unterhalb von -78,5° C liegt um ein Vereisen des Desinfektionsmittels D zu vermeiden, was jedoch nicht zwingend notwendig ist.

Das Verfahren ist besonders zum Reinigen von Gegenständen geeignet, die regelmäßig nacheinander von verschiedenen Menschen gebraucht werden sollen und daher insbesondere in einer Krankenhausumgebung laufend desinfiziert werden müssen, wie z.B. Krankenhausbetten sowie sonstiges Patientenmobiliar und andere Geräte, um die Verbreitung von Keimen zu vermeiden.

Die besagte Düse 2 bildet einen Endabschnitt eines Strömungspfades 20 der Vorrichtung 1, wobei der Strömungspfad 20 abschnittsweise durch einen mit der Düse 2 verbundenen flexiblen Schlauch gebildet sein kann, der ein Ausrichten der Düse 2 auf den Gegenstand 10 erlaubt. Die Düse 2 kann in eine Strahlpistole integriert sein, wobei die Strahlpistole von einer Person greifbar und dabei händisch führbar ist, so dass die Düse 2 durch die Person auf die zu reinigende/desinfizierende Oberfläche 11 gerichtet werden kann.

Die Vorrichtung 1 kann gemäß Figur 1 insbesondere dazu ausgebildet sein, das Trockeneis T in Form einer Vielzahl an Trockeneiskörpern (z.B. Pellets) in das Trägergas G zu geben. Hierzu kann die Vorrichtung 1 einen Behälter 5 zum Speichern der Trockeneiskörper/Pellets T aufweisen, der mit dem Strömungspfad 20 verbindbar ist, z.B. über eine Schleuse 50, so dass das Trockeneis T über die Schleuse 50 in den Strömungspfad 20 einleitbar sind und damit dem Trägergas T zuführbar sind. Der Behälter kann in einem verfahrbaren Wagen bzw. Gehäuse 51 der Vorrichtung 1 angeordnet sein, wobei der besagte Schlauch 20 an das Gehäuse 51 angeschlossen ist. Die Verfahrbarkeit des Gehäuses 51 gestattet es die Düse um den Gegenstand 10 herum zu führen und erlaubt somit auch das Reinigen/Desinfizieren von größeren Gegenständen 10.

Alternativ kann die Vorrichtung 1 einen Behälter zum Speichern von Kohlendioxid in flüssiger Form aufweisen, wobei die Vorrichtung 1 dann dazu ausgebildet ist, flüssiges Kohlendioxid unter Erzeugung von Trockeneisschnee zu expandieren und sodann dem Strömungspad 20 bzw. dem darin strömenden Trägergas G zuzugeben.

Weiterhin weist der Strömungspfad 20 vorzugsweise eine Einspeisestelle 4.1 auf (z. B. einen sogenannten Öler), über die das Desinfektionsmittel D im flüssigen Zustand in den Strömungspfad 20 bzw. in das darin geführte Trägergas G einleitbar ist. Ein Volumenstrom des Trägergases G kann mit einem Ventil 6 reguliert werden. Weiterhin kann stromab des Ventils 6 ein Filter 3 zum Filtern des Trägergases G vorgesehen sein.

Vorzugsweise wird das Trockeneis T dabei stromab der Einspeisestelle 4.1 für das Desinfektionsmittel D in den Strömungspfad 20 bzw. in das darin geführte Trägergas G gegeben, d.h. vorzugsweise stromauf des Gehäuses/Wagens 51. Hierzu gibt es jedoch auch alternative Anordnungen (siehe oben). So kann z.B. das Trägergas G alternativ über eine Einspeisestelle 4.2 in das Trägergas G eingeleitet werden, die stromab der Einspeisung des Trockeneises liegt, insbesondere stromab der Schleuse 50.

Das Einleiten des Desinfektionsmittels D stromauf des Gehäuses/Wagens 51 erlaubt es mit Vorteil bestehende Strahlanlagen bzw. Vorrichtungen 1 zu verwenden, die somit auf einfache Weise mit der Einspeisestelle 4.1 bzw. 4.2 nachgerüstet werden können.

Die Einspeisestelle 4 kann gemäß dem Detail der Figur 1 eine Venturidüse 40 aufweisen, wobei das Desinfektionsmittel D über die Venturidüse 40 in den Strömungspfad 20 bzw. das darin geführte Trägergas G gegeben wird. Hierzu kann eine Zuleitung 41 vorgesehen sein, die strömungstechnisch mit einer Engstelle 42 der Venturidüse 40 verbunden ist. Stromauf der Engstelle 42 kann ein Drosselventil 43 zum Drosseln eines Volumenstroms des Desinfektionsmittels D vorgesehen sein. Das Drosselventil 43 kann so ausgestaltet sein, dass es bei Bedarf geschlossen werden kann, um Reinigung ohne Zugabe von Desinfektion zu ermöglichen.

Die vorliegende Erfindung erlaubt mit Vorteil eine Herstellung einer Mischung aus CO₂-Pellets T und einem Desinfektionsmittel D und erspart somit Zeit und Kosten. Diese Zusammensetzung kann mittels eines Trägergases G auf einfache Weise beschleunigt bzw. transportiert werden und kann somit auf die zu reinigende/desinfizierende Oberfläche über eine Düse gestrahlt werden. Hierdurch können die Reinigung sowie die Desinfektion in einem Arbeitsgang durchgeführt werden, wobei die CO₂-Pellets/Körper oder der CO₂-Schnee rückstandsfrei sublimieren. Das mitgeführte Desinfektionsmittel D wird auf der Oberfläche des Gegenstands noch einige Zeit vorliegen, bevor es ebenfalls verdampft ist. Je nach Siedepunkt des Desinfektionsmittels ist diese Zeit lange genug, so dass das Desinfektionsmittel seine Wirkung entfalten kann. Die kühlende Wirkung des Trockeneises verlängert weiterhin die Zeit bis zum Verdampfen des Desinfektionsmittels und begünstigt damit die desinfizierende Wirkung.

Die Figur 2 zeigt eine alternative Ausführungsform eines erfindungsgemäßen Verfahrens bzw. einer erfindungsgemäßen Vorrichtung 1. Auch hier werden in vorteilhafter Weise zwei bisher unabhängig voneinander eingesetzte Verfahren zur Reinigung und Desinfektion von Oberflächen verwendet, wobei beide Verfahren mit Vorteil die spezifische Eigenschaft aufweisen, dass keine Flüssigkeiten eingesetzt werden und das Verfahren somit ein Trockenverfahren darstellt.

Die zu reinigenden Produkte können wiederum z.B. im Bereich der Medizin (Einrichtungsgegenstände, Medizingeräte, etc.), Lebensmittel, Pharmazie (z.B. Produktionsanlagen) vorliegen (siehe oben).

Insbesondere kann die erfindungsgemäße Vorrichtung eine Düse 2 (z.B. in Form einer Trockeneisstrahlpistole) aufweisen, an der eine UV-Lampe 7, insbesondere eine UV-C-Lampe 7, angebracht ist, die in Richtung des Trockeneisstrahls bzw. des Stoffstroms S UV-Licht 8 (vorzugsweise UV-C-Licht) abstrahlt. Von UV-C-Licht spricht man insbesondere, wenn die Wellenlänge des Lichts im Bereich von 100 nm bis 280 nm liegt. Neben der Reinigung des Gegenstands mit Hilfe von Trockeneis (z.B. Micropellets) wird bereits durch die Trockeneisreinigung selbst ein entkeimender Effekt festgestellt. Um den Desinfektionsgrad jedoch zu optimieren, kommt die gleichzeitige Bestrahlung mit UV-Licht bzw. UV-C Licht zur Anwendung. Insofern findet Reinigung und Desinfektion vorzugsweise in einem Arbeitsschritt statt, da zum Zeitpunkt der Reinigung mit dem Stoffstrom S bzw. dem darin enthaltenen Trockeneis eine gleichzeitige und ergänzende Desinfektion stattfindet.

Durch Wahl der Einstellparameter kann der Bediener den Grad der Reinigung bzw. Desinfektion an die Anforderungen anpassen. Das Betrifft z.B. den Strahldruck, die Menge an Trockeneis und die Größe der Trockeneis-Strahlpartikel (beispielsweise von 0,1 mm bis 3 mm).

Im Einzelnen kann bei der Ausführungsform gemäß Figur 2 zum Reinigen eines Gegenstands 10 ein Stoffstrom S erzeugt werden, der Trockeneis T und ein Trägergas G aufweist, wobei das Trägergas (z.B. Druckluft) zum Beschleunigen des Trockeneises verwendet wird. Dieser Stoffstrom S wird aus einer Düse 2 der Vorrichtung 1 ausgestoßen, die z.B. wiederum als Lavaldüse ausgebildet sein kann. Der Stoffstrom S trifft sodann auf eine Oberfläche 11 des Gegenstands 10, wobei die Oberfläche 11 mittels des darauf auftreffenden Trockeneises T gereinigt wird. Die zusätzliche Bestrahlung mit dem UV-Licht 8 über zumindest eine UV-Lampe, die bezüglich der Düse 2 festgelegt sein kann, sorgt für die Desinfektion der Oberfläche 11.

Die Düse 2 kann wiederum einen Endabschnitt des Strömungspfades 20 der Vorrichtung 1 bilden, wobei der Strömungspfad 20 abschnittsweise durch einen mit der Düse 2 verbundenen flexiblen Schlauch gebildet sein kann, der ein Ausrichten der Düse 2 auf den Gegenstand 10 erlaubt.

Die Vorrichtung 1 kann gemäß Figur 2 insbesondere dazu ausgebildet sein, das Trockeneis T in Form einer Vielzahl an Trockeneiskörpern (z.B. Pellets) in das Trägergas G zu geben. Hierzu kann die Vorrichtung 1 einen Behälter 5 zum Speichern der Trockeneiskörper/Pellets T aufweisen, der mit dem Strömungspfad 20 verbindbar ist, z.B. über eine Schleuse 50, so dass das Trockeneis T über die Schleuse 50 in den Strömungspfad 20 einleitbar sind und damit dem Trägergas T zuführbar sind. Der Behälter kann wie zuvor in einem verfahrbaren Wagen bzw. Gehäuse 51 der Vorrichtung 1 angeordnet sein, wobei der besagte Schlauch 20 an das Gehäuse 51 angeschlossen ist. Die Verfahrbarkeit des Gehäuses 51 gestattet es wiederum, die Düse um den Gegenstand 10 herum zu führen und erlaubt somit auch das Reinigen/Desinfizieren von größeren Gegenständen 10.

Auch die weitere Ausführungsform gemäß Figur 2 weist die Vorteile auf, wonach die Reinigung und die Desinfektion in nur einem Arbeitsschritt erfolgt. Weiterhin handelt es sich ebenfalls um ein trockenes Verfahren: Die gereinigten und desinfizierten Geräte können direkt wieder eingesetzt werden, ohne zeitaufwändige Trocknungsphase. Dies unterstützt den spezifischen Vorteil von Trockeneisreinigungsverfahren. Weiterhin sind keine Chemikalien notwendig, so dass insofern ein umweltschonendes Verfahren vorliegt.

## Patentansprüche

1. Verfahren zum Reinigen und Desinfizieren eines Gegenstands (10), aufweisend die Schritte:
- Erzeugen eines Stoffstromes (S) aufweisend Trockeneis (T) und ein Trägergas (G), und
- Ausstoßen des Stoffstromes (S) aus einer Düse (2), so dass der Stoffstrom (S) auf eine Oberfläche (11) des Gegenstands (10) auftrifft, wobei die Oberfläche (11) mittels des Trockeneises (T) gereinigt wird und mittels eines Mittels (D, 8) zum Desinfizieren (D, 8) desinfiziert wird.

2. Verfahren nach Anspruch 1, wobei das Mittel zum Desinfizieren ein Desinfektionsmittel ist, wobei der Stoffstrom (S) das Desinfektionsmittel aufweist.

3. Verfahren nach Anspruch 1 oder 2, wobei das Desinfektionsmittel einen der folgenden Stoffe aufweist oder durch einen der folgenden Stoffe gebildet ist: Methanol, Ethanol, Propanol, Butanol, Pentanol, Hexanol, Heptanol, Oktanol, Isopropanol, Isobutanol, Isopentanol, Ethylenglykol, 1,3-Propylenglykol, und/oder wobei wobei das Desinfektionsmittel (D) einen Gefrierpunkt aufweist, der unterhalb von -78,5° C liegt.

4. Verfahren nach einem der vorhergehenden Ansprüche, wobei das Trockeneis (T) in Form einer Vielzahl an Trockeneiskörpern in das Trägergas (G) gegeben wird.

5. Verfahren nach einem der Ansprüche 1 bis 3, wobei das Trockeneis (T) in Form von Trockeneisschnee in das Trägergas (G) gegeben wird.

6. Verfahren nach einem Anspruch 5, wobei Kohlendioxid in flüssiger Form vorgehalten wird, in einer Expansionskammer unter Entstehung des Trockeneisschnees expandiert wird, der sodann in das Trägergas (G) gegeben wird.

7. Verfahren nach einem der vorhergehenden Ansprüche, wobei das Desinfektionsmittel (D) im flüssigen Zustand an einer Einspeisestelle (4) in das Trägergas (G) gegeben wird, wobei insbesondere das Trockeneis (T) stromab der Einspeisestelle (4) in das Trägergas (G) gegeben wird, oder wobei insbesondere das Trockeneis (T) stromauf der Einspeisestelle (4.1) in das Trägergas (G) gegeben wird.

8. Verfahren nach einem der vorhergehenden Ansprüche, wobei das Desinfektionsmittel (D) über ein Drosselventil (43) und eine Venturidüse (40) in das Trägergas (G) gegeben wird.

9. Verfahren nach einem der Ansprüche 1 bis 5, wobei das Desinfektionsmittel (D) dem Trockeneis (T) zugegeben wird, bevor das Trockeneis (T) in das Trägergas (G) gegeben wird, und/oder wobei zumindest ein Trockeneisblock aufweisend das Desinfektionsmittel (D) bereitgestellt wird, wobei der Trockeneisblock zur Erzeugung der Trockeneiskörper zerkleinert wird (z.B. mittels eines Mahlwerks oder eines sonstigen Zerkleinerers)

10. Verfahren nach einem der vorhergehenden Ansprüche, wobei bezogen auf eine Menge Trockeneis (T) 0,5 Gew.-% bis 10 Gew.-% Desinfektionsmittel (D), vorzugsweise 1 Gew.-% bis 5 Gew.-%. Desinfektionsmittel (D), der Menge an Trockeneis (T) zugesetzt wird.

11. Verfahren nach Anspruch 1, wobei das Mittel zum Desinfizieren UV-Licht (8) ist, wobei die Oberfläche (11) vor und/oder während und/oder nach dem Reinigen der Oberfläche (11) mittels des Trockeneises (T) mit dem UV-Licht (8) beaufschlagt wird, um die Oberfläche (11) zu desinfizieren.

12. Verfahren nach einem der vorhergehenden Ansprüche, wobei es sich bei dem Gegenstand (10) um einen der folgenden Gegenstände (10) handelt: ein Bett, insbesondere ein Krankenhausbett, eine Patientenliege, ein Kommode, ein Nachttisch, ein Therapiegerät, ein elektronisches Gerät, ein Radio, ein Fernseher, ein Bedientableau, ein Patientenstuhl, ein Patiententisch, ein Gasbehälter.

13. Verfahren nach einem der vorhergehenden Ansprüche, wobei das Trägergas (G) eines der folgenden Gase ist oder eines der folgenden Gase aufweist: Luft, gasförmiger Stickstoff, gasförmiges Kohlendioxid.

14. Vorrichtung (1) zum Reinigen und Desinfizieren eines Gegenstands (10), wobei die Vorrichtung (1) dazu ausgebildet ist, einen Stoffstrom (S) aufweisend Trockeneis (T) und ein Trägergas (G) bereitzustellen, wobei die Vorrichtung (1) weiterhin dazu ausgebildet ist ein Mittel zum Desinfizieren (D) bereitzustellen, wobei die Vorrichtung (1) weiterhin eine Düse (2) aufweist und dazu ausgebildet ist, den Stoffstrom (S) aus der Düse (2) auszustoßen.

15. Vorrichtung nach Anspruch 14, wobei das Mittel zum Desinfizieren ein Desinfektionsmittel ist (D), das der Stoffstrom (S) aufweist, oder wobei das Mittel zum Desinfizieren UV-Licht (8) ist, wobei die Vorrichtung (1) zumindest eine UV-Lampe (7) zum Erzeugen des UV-Lichts (8) aufweist.
